# EUROPÄISCHE PATENTSCHRIFT

(11) **EP 1 860 954 B1**
(45) Veröffentlichungstag und Bekanntmachung des Hinweises auf die Patenterteilung: **25.01.2012**
(21) Anmeldenummer: 05803021.4
(22) Anmeldetag: 15.11.2005
(51) Int. Cl.: A23K 1/00, A23L 1/015

(54) **MIKROORGANISMUS ZUR ENTGIFTUNG VON FUMONISINEN SOWIE VERWENDUNG DESSELBEN, VERFAHREN ZUM ENTGIFTEN VON FUMONISINEN UND FUTTERMITTELZUSATZ, ENTHALTEND DEN MIKROORGANISMUS**
MICRO-ORGANISM FOR DECONTAMINATING FUMONISINS AND ITS USE, METHOD FOR DECONTAMINATING FUMONISINS AND FEED ADDITIVES CONTAINING SAID MICRO-ORGANISM
MICRO-ORGANISME POUR DECONTAMINER DE FUMONISINES ET SON UTILISATION, PROCEDE POUR DECONTAMINER DE FUMONISINES ET ADDITIF D'ALIMENT POUR ANIMAUX CONTENANT CE MICRO-ORGANISME

(30) Priorität: 16.11.2004 AT 19122004
(43) Veröffentlichungstag der Anmeldung: 05.12.2007
(73) Patentinhaber: Erber Aktiengesellschaft, 3130 Herzogenburg (AT)
(72) Erfinder: SCHATZMAYR, Gerd, A-1190 Wien (AT); TÄUBEL, Martin, A-2630 Ternitz (AT); VEKIRU, Elisavet, A-1150 Wien (AT); BINDER, Eva-Maria, A-7501 Unterwart 338 (AT)
(74) Vertreter: Cunow, Gerda
(86) Internationale Anmeldenummer: PCT/AT2005/000453
(87) Internationale Veröffentlichungsnummer: WO 2006/053357

(56) Entgegenhaltungen:
- WO-A-96/12414
- WO-A-2004/085624
- US-A1- 2004 208 956
- US-B1- 6 482 621
- US-B1- 6 514 749
- MOLL W -D ET AL: "Isolation and characterization of microorganisms for the biological inactivation of fumonisins" JOURNAL OF BIOTECHNOLOGY, Bd. 118, Nr. Suppl. 1, August 2005 (2005-08), Seiten S183-S184, XP002400852 & 12TH EUROPEAN CONGRESS ON BIOTECHNOLOGY (ECB 12); COPENHAGEN, DENMARK; AUGUST 21 -24, 2005 ISSN: 0168-1656
- CAVAGLIERI LILIA RENEE ET AL: "Rhizobacteria and their potential to control Fusarium verticillioides: effect of maize bacterisation and inoculum density" ANTONIE VAN LEEUWENHOEK, Bd. 87, Nr. 3, April 2005 (2005-04), Seiten 179-187, XP002400853 ISSN: 0003-6072

## Beschreibung

Die vorliegende Erfindung bezieht sich auf einen Mikroorganismus zur Entgiftung von Fumonisinen und Fumonisin-Derivaten sowie auf die Verwendung von Bakterien oder Hefen, alleine oder in Kombination von zwei oder mehreren Stämmen zur Detoxifizierung von Fumonisinen und Fumonisin-Derivaten in Nahrungsmitteln und/oder Futtermitteln, ein Verfahren zur Entgiftung von Fumonisinen und Fumonisin-Derivaten unter Verwendung eines Mikroorganismus sowie auf einen Futtermittelzusatz zur Inaktivierung von Mykotoxinen, insbesondere Fumonisinen und Fumonisin-Derivaten.

Mykotoxine, welche eine Vielzahl von verschiedensten Toxinen umfassen, stellen in der modernen Nahrungsmittel- und Futtermittelindustrie ein zunehmendes Problem dar, da eine Vielzahl von Pflanzen, welche nachfolgend zu Nahrungsmitteln bzw. zu Futtermitteln verarbeitet werden bzw. direkt Tieren verfüttert werden, mit den unterschiedlichsten Toxinen in unterschiedlichsten Konzentrationen befallen sind, so dass neben der Tatsache, dass das jeweilige Toxin nachgewiesen werden muss, ein effizientes und unschädliches Verfahren zur Entgiftung bzw. zum Abbau der entsprechenden Toxine herangezogen werden muss bzw. gefunden werden muss.

Ein Weg, toxinfreie Pflanzen zu erhalten, ist der Versuch, sogenannte transgene Pflanzen zu züchten, welche gegenüber bestimmten Toxinen resistent sind oder durch Einsatz von "genmanipulierten" Pflanzen zu Nahrungsmitteln zu gelangen, welche aufgrund der Resistenz der entsprechenden Pflanzen gegenüber den Toxinen frei von denselben sind.

Dieser Weg ist neben der Tatsache, dass er äußerst komplex und kompliziert ist, in vielen Ländern der Erde nicht unumstritten und es wird versucht, eine andere Art der Entgiftung von Pflanzen zu finden.

Der WO 96/12414 ist ein Futtermittelzusatz zur Inaktivierung von Mycotoxinen entnehmen, bei welchem einem Futtermittel ein aus Enzymen produzierenden Organismen hergestelltes Enzympräparat in einem Futtermittel enthalten ist, wobei die Enzymen produzierenden Organismen aus dem Gruppen, die zur Bindung von Epoxidasen und/oder Lactonasen befähigt sind, gewählt sind.

Der WO 2004/085624 sind Transaminasen, Deaminasen und Aminomuthasen und Zusammensetzungen davon, sowie Verfahren zur enzymatischen Detoxifizierung von aminierten Toxinen, beispielsweise Mycotoxinen, wie Fumonisienen entnehmbar, bei welchen die Gene für die Enzyme direkt aus der Umwelt isoliert wurden.

Der US 2004/0208956 ist ein Mikroorganismus für die biologische Inaktivierung oder Detoxifizierung von Mycotoxinen, insbesondere Ochratoxin, welche im Minimalmedium detoxifizieren können.

Der US-A 6,482,511 sind Zusammensetzungen aus Verfahren zur vollständigen Detoxifizierung von Fumonisinen mittels isolierter und definierter Enzyme entnehmbar.

Weiterhin ist der US 6,514,749 B1 ein Verfahren zum Identifizieren von Organismen entnehmbar, welche fähig sind, Fumonisin abzubauen, wobei diese Organismen zum Isolieren der Enzymengene, welche für das Verleihen einer Fumonisinresistenz verantwortlich sind, verwendet werden und die Gene nachfolgend beispielsweise in Pflanzen oder Mikroorganismen der Rumenflora zur Expression zu bringen.

Toxine, welche insbesondere auf Mais häufig anzutreffen sind und zu schweren Beeinträchtigungen nach Verzehr desselben führen, sind Fumonisine bzw. die Fumonisin-Derivate, welche zwar in Laborversuchen durch bereits bekannte Mikroorganismen abgebaut werden können, für welche jedoch bis dato keine Mikroorganismen gefunden wurden, welche einen derartigen Abbau in unterschiedlichsten Konzentrationen des Toxins und in unterschiedlichen Nährumgebungen durchführen können. Darüber hinaus benötigen bekannte Mikroorganismen für diesen Abbau nicht unbeträchtliche Zeitspannen von mehr als 24 h, so dass derartige Mikroorganismen in einem gewerblichen bzw. Handelsmaßstab nicht sinnvoll eingesetzt werden können.

Ein weiteres Problem im Zusammenhang mit Mykotoxinen bei Nahrungs- und Futtermitteln ist, dass aufgrund der Tatsache, dass immer mehr Mischfuttermittel bzw. Nahrungsmittel, welche eine Mehrzahl von Getreide bzw. Getreidesorten enthalten, hergestellt werden, gleichzeitig mehrere Mykotoxine in ein und desselben Nahrungs- bzw. Futtermittel vorkommen und dass ein sinnvoller bzw. gezielter Abbau derselben erforderlich scheint. Darüber hinaus wurde in neuerlichen Studien festgestellt, dass die Toxine untereinander kombinatorische Wechselwirkungen zeigen können, welche die schädliche Wirkung der einzelnen Toxine weiter verstärken. So wurde beispielsweise von Harvey 1996 berichtet, dass es zu synergistischen Effekten von Fumonisinen und Deoxynivalenol bei Schweinen kommt. Zusätzlich wird angenommen, dass die Mehrzahl von Toxinen, welche beispielsweise in Futtermitteln enthalten sein können, zu immunsuppressiven Effekten bei Tieren führen, die auf das parallele Auftreten von Mykotoxinen zurückgeführt werden.

Die vorliegende Erfindung zielt nun darauf ab, Mikroorganismen zur Entgiftung von Fumonisinen und Fumonisin-Derivaten zur Verfügung zu stellen, welche einerseits das Toxin extrem rasch abbauen können und andererseits neben dem raschen Abbau den Abbau auch in Anwesenheit unterschiedlichster Nährstoffkonzentrationen durchführen können. Schließlich zielt die vorliegende Erfindung darauf ab, einen Mikroorganismus bzw. Kombinationen von Mikroorganismen zur Verfügung zu stellen, welche(r) neben Fumonisinen noch weitere Toxine allein oder in Kombination abbauen kann (können), um insbesondere bei Einsatz unterschiedlichster Futterpflanzen zu einem toxinfreien Futtermittel zu gelangen.

Zur Lösung dieser Aufgaben wird gemäß der vorliegenden Erfindung ein Mikroorganismus zur Entgiftung von Fumonisinen und Fumonisin-Derivaten zur Verfügung gestellt, wobei detoxifizierende Bakterien oder Hefen, gewählt aus den Stämmen DSM 16254 und DSM 16257, zuordenbar dem Taxon Sphingomonadaceae, dem Stamm DSM 16255, zuordenbar dem Taxon Rhizobiales, dem Stamm DSM 16256, zuordenbar dem Taxon Microbacteriaceae, dem Stamm DSM 16253, zuordenbar dem Taxon Rhizobiaceae, dem Stamm DSM 16252, zuordenbar dem Taxon Alcaligenaceae und Pichia sp. DSM 16562, eingesetzt sind, welche Fumonisine enzymatisch in einer mehrstufigen Reaktion in deaminierte Metaboliten umwandeln. Die obengenannten MikroOrganismen sind nicht nur in der Lage, Fumonisine enzymatisch in einer mehrstufigen Reaktion in deaminierte Metaboliten umzuwandeln, sondern können dies in extrem kurzen Zeiträumen und auch in Anwesenheit komplexer Umgebungen, wie beispielsweise oder Nahrungsmitteln, d.h. in Anwesenheit mehrerer bzw. unterschiedlichster Kohlenstoffquellen und insbesondere bei Anwesenheit eines Nährstoffüberangebots.

Im Einzelnen können die Mikroorganismen wie folgt kurz beschrieben werden. Stamm DSM 16254 ist nach partieller Sequenzierung der 16S rDNA mit dem Primer 27 forward (Sequenzlänge 689 bp) dem Taxon Sphingomonadaceae zuzuordnen. Die partielle 16S rDNA Sequenz weist folgende Basenabfolge auf: 
1 GAACGCTGGS GGCATGCCTA ACACATGCAA GTCGAACGAA GTCTTCGGAC TTAGTGGCGC
61 ACGGGTGCGT AACGCGTGGG AATCTGCCCT TGGGTACGGA ATAACTCAGA GAAATTTGTG
121 CTAATACCGT ATAATGTCTT CGGACCAAAG ATTTATCGCC CAAGGATGAG CCCGCGTAAG
181 ATTAGCTAGT TGGTGGGGTA AAGGCCCACC AAGGCGACGA TCTTTAGCTG GTCTGAGAGG
241 ATGATCAGCC ACACTGGGAC TGAGACACGG CCCAGACTCC TACGGGAGGC AGCAGTGGGG
301 AATATTGGAC AATGGGCGAA AGCCTGATCC AGCAATGCCG CGTGAGTGAT GAAGGCCCTA
361 GGGTTGTAAA GCTCTTTTAC CCGGGATGAT AATGACAGTA CCGGGAGAAT AAGCTCCGGC
421 TAACTCCGTG CCAGCAGCCG CGGTAATACG GAGGGAGCTA GCGTTGTTCG GAATTACTGG
481 GCGTAAAGCG CGCGTAGGCG GTTTTTCAAG TCAGAGGTGA AAGCCCGGGG CTCAACCCCG
541 GAATTGCCTT TGAAACTGGA AGACTTGAAT CTTGGAGAGG TCAGTGGAAT TCCGAGTGTA
601 GAGGCGAAAT TCGTAGATAT TCGGAAGAAC ACCAGTGGCG AAGGCGACTG ACTGGACAAG
661 ATTGACGCTG AGGTGCGAAA GCGTGGGGA
 wobei der Mikroorganismus Gram negativ ist, kleine Stäbchen bildet, die überwiegend in Einzelzellen vorkommen und teilweise filamentöse Kettenstrukturen ausbilden.

Stamm DSM 16257 gehört nach partieller Sequenzierung der 16S rDNA (mit Primer 30 reverse, erzielte Sequenzlänge 426 bp) ebenfalls dem Taxon Sphingomonadaceae an. Es ergibt sich folgende Sequenz:

1 GATCCTGGCT CAGAACGAAC GCTGGCGGCA TGCCTAACAC ATGCAAGTCG AACGAAGTCT
61 TCGGACTTAG TGGCGCACGG GTGCGTAACG CGTGGGAATC TGCCCTTGGG TACGGAATAA
121 CTCAGAGAAA TTTGTGCTAA TACCGTATAA TGACTTCGGT CCAAAGATTT ATCGCCCAAG
181 GATGAGCCCG CGTAAGATTA GCTAGTTGGT GGGGTAAAAG CCTACCAAGG CGACGATCTT
241 TAGCTGGTCT GAGAGGATGA TCAGCCACAC TGGGACTGAG ACACGGCCCA GACTCCTACG
301 GGAGGCAGCA GTGGGGAATA TTGGACAATG GGCGAAAGCC TGATCCAGCA ATGCCGCGTG
361 AGTGATGAAG GCCCTAGGGT TGTAAAGCTC TTTTACCCGG GATGATAATG ACAGTACCGG
421 GAGAAT

Dieser Mikroorganismus bildet kleine Stäbchen, die zum größten Teil in langen, filamentösen Zellstrukturen angeordnet sind.

DSM 16255 liefert nach partieller Sequenzierung der 16S rDNA mit Primer 27 forward die folgende, 720 bp lange Sequenz:

1 ACGCTGGCGG CAGGCTTAAC ACATGCAAGT CGAACGGTCT CTTCGGAGGC AGTGGCAGAC
61 GGGTGAGTAA TGCATGGGAA TCTACCGTTC TCTACGGAAT AACTCAGGGA AACTTGTGCT
121 AATACCGTAT ACGCCCTTTT GGGGAAAGAT TTATCGGAGA ATGATGAGCC CATGTTGGAT
181 TAGCTAGTTG GTAGGGTAAA GGCCTACCAA GGCGACGATC CATAGCTGGT CTGAGAGGAT
241 GATCAGCCAC ACTGGGACTG AGACACGGCC CAGACTCCTA CGGGAGGCAG CAGTGGGGAA
301 TATTGGACAA TGGGCGCAAG CCTGATCCAG CCATGCCGCG TGAGTGATGA AGGCCCTAGG
361 GTTGTAAAGC TCTTTCACCG GTGAAGATAA TGACGGTAAC CGGAGAAGAA GCCCCGGCTA
421 ACTTCGTGCC AGCAGCCGCG GTAATACGAA GGGGGCTAGC GTTGTTCGGA TTTACTGGGC
481 GTAAAGCGCA CGTAGGCGGA CTTTTAAGTC AGGGGTGAAA TCCCGGGGCT CAACCCCGGA
541 ACTGCCTTTG ATACTGGAAG TCTTGAGTAT GGAAGAGGTA AGTGGAATTG CGAGTGTAGA
601 GGTGAAATTC GTAGATATTC GCAGGAACAC CAGTGGCGAA GGCGGCTTAC TGGTCCATTA
661 CTGACGCTGA GGTGCGAAAG CGTGGGGAG CAAACAGGAT TAGATACCCT GGTAGTCCAC

Der Mikroorganismus gehört dem Taxon Rhizobiales an, ist Gram negativ und bildet kleine Stäbchen überwiegend in Einzelzellen.

Der Mikroorganismus DSM 16256 ist dem Taxon Microbacteriaceae zuordenbar. Die partielle Sequenzierung der 16S rDNA mit Primer 27 forward liefert folgende, 706 bp lange Sequenz:

1 GAACGCTGGC GGCGTGCTTA ACACATGCAA GTCGAACGAT GAAGCTGGAG CTTGCTCTGG
61 TGGAAGAGTG GCGAACGGGT GAGTAACACG TGAGTAACCT GCCCCAGACT CTGGGATAAG
121 CGCTGGAAAC GGCGTCTAAT ACTGGATATG ACCCCTACAG GCATCTGTTG GGGGTGGAAA
181 GATTTATCGG TCTGGGATGG GCTCGCGGCC TATCAGCTAG ATGGTGAGGT AACGGCTCAC
241 CATGGCGACG ACGGGTAGCC GGCCTGAGAG GGTGACCGGC CACACTGGGA CTGAGACACG
301 GCCCAGACTC CTACGGGAGG CAGCAGTGGG GAATATTGCA CAATGGGCGA AAGCCTGATG
361 CAGCAACGCC GCGTGAGGGA TGACTGCCTT CGGGTTGTAA ACCTCTTTTA GTAGGGAAGA
421 AGCGAAAGTG ACGGTACCTG CAGAAAAAGC ACCGGCTAAC TACGTGCCAG CAGCCGCGGT
481 AATACGTAGG GTGCAAGCGT TGTCCGGAAT TATTGGGCGT AAAGAGCTCG TAGGCGGCTT
541 GTCGCGTCTG CTGTGAAAAC CCGAGGCTCA ACCTCGGGCC TGCAGTGGGT ACGGGCAGGC
601 TAGAGTGCGG TAGGGGAGAT TGGAATTCCT GGTGTAGCGG TGGAATGCGC AGATATCAGG
661 AGGAACACCG ATGGCGAAGG CAGATCTCTG GGCCGCTACT GACGCT

Der Mikroorganismus ist Gram positiv und weist kleine kurze Stäbchen, die teilweise zu kettigen Zellverbänden angeordnet sind, auf.

DSM 16253 gehört nach partieller Sequenzierung der 16S rDNA (Primer 530 reverse, Sequenzlänge 392 bp) dem Taxon Rhizobiaceae an. Die Sequenz lautet wie folgt:

1 TCCTGGCTCA GAACGAACGC TGGCGGCAGG CTTAACACAT GCAAGTCGAG CGCCCCGCAA

61 GGGGAGCGGC AGACGGGTGA GTAACGCGTG GGAATCTACC GAGCCCTGCG GAATAGCTCC

121 GGGAAACTGG AATTAATACC GCATACGCCC TACGGGGGAA AGATTTATCG GGGTTTGATG

181 AGCCCGCGTT GGATTAGCTA GTTGGTGGGG TAAAGGCCTA CCAAGGCGAC GATCCATAGC

241 TGGTCTGAGA GGATGATCAG CCACATTGGG ACTGAGACAC GGCCCAAACT CCTACGGGAG

301 GCAGCAGTGG GGAATATTGG ACAATGGGCG CAAGCCTGAT CCAGCCATGC CGCGTGAGTG

361 ATGAAGGCCC TAGGGTTGTA AAGCTCTTTC AC

Der Mikroorganismus ist Gram negativ und weist kleine Stäbchen auf, welche überwiegend als Einzelzellen vorkommen.

Der Mikroorganismus DSM 16252 ist dem Taxon Alcaligenaceae zuordenbar. Die partielle Sequenzierung der 16S rDNA lie-fert ein 476 bp langes DNA-Framgent mit folgender Nukleotidsequenz:

1 TCCTGGCTCA GATTGAACGC TAGCGGGATG CCTTACACAT GCAAGTCGAA CGGCAGCACG

61 GACTTCGGTC TGGTGGCGAG TGGCGAACGG GTGAGTAATG TATCGGAACG TGCCTAGTAG

121 CGGGGGATAA CTACGCGAAA GCGTAGCTAA TACCGCATAC GCCCTACGGG GGAAAGCAGG

181 GGATCGCAAG ACCTTGCACT ATTAGAGCGG CCGATATCGG ATTAGCTAGT TGGTGGGGTA

241 ACGGCTCACC AAGGCGACGA TCCGTAGCTG GTTTGAGAGG ACGACCAGCC ACACTGGGAC

301 TGAGACACGG CCCAGACTCC TACGGGAGGC AGCAGTGGGG AATTTTGGAC AATGGGGGAA

361 ACCCTGATCC AGCCATCCCG CGTGTGCGAT GAAGGCCTTC GGGTTGTAAA GCACTTTTGG

421 CAGGAAAGAA ACGTCATGGG CTAATACCCC GTGAAACTGA CGGTACCTGC AGAATA

Der Mikroorganismus ist Gram negativ und weist kleine, gerade Stäbchen auf, die teilweise in klumpigen Vielzellverbänden vorkommen, auf.

DSM 16562, nämlich Pichia sp., zeigt ovale, relativ kleine Hefezellen, die einzeln und nicht in Zellverbänden vorkommen.

Im einzelnen konnte nachgewiesen werden, dass, obwohl die Mikroorganismen untereinander relativ unterschiedlich sind, sie neben der Tatsache, dass sie extrem rasch detoxifizieren können, alle gemeinsam die Eigenschaft besitzen, diese Detoxifizierung von Fumonisinen auch in komplexen Umgebungen rasch und zuverlässig auszuführen.

Gemäß einer Weiterbildung der Erfindung sind die Bakterien oder Hefen in Form von Pulver, Flüssigkeiten oder Gel stabilisiert, wodurch ein stabiles, jederzeit für den jeweiligen Einsatz anwendbares Produkt zur Verfügung gestellt wird.

Um möglichst vollständig Toxine aus Nahrungs- bzw. Futtermitteln zu entferrien, ist es gemäß einer Weiterbildung der Erfindung möglich, mit den Mikroorganismen der vorliegenden Erfindung neben Fumonisin und Fumonisin-Derivaten wenigstens ein weiteres Mykotoxin, gewählt aus Zearalenonen, Aflatoxinen oder, zu detoxifizieren. Im einzelnen hat sich herausgestellt, dass die erfindungsgemäßen Mikroorganismen zumindest ein weiteres Toxin detoxifizieren können, wobei Detoxifizierung ebenso rasch und effizient erzielbar ist wie die Detoxifizierung von Fumonisinen. Dadurch gelingt es durch Bereitstellen der Mikroorganismen ohne weiteren Zusatz, eine Mehrzahl von Toxinen in einem Nahrungs- bzw. Futtermittel, insbesondere in einer Nahrungs- bzw. Füttermittelmischung, vollständig abzubauen und somit ein hochwertiger, toxinfreies Nahrungs- bzw. Futtermittel zur Verfügung zu stellen.

Die Erfindung betrifft auch die Verwendung von Bakterien oder Hefen, alleine oder in Kombination von zwei oder mehreren Stämmen, gewählt aus dem Stamm DSM 16254 und DSM 16257, zuordenbar dem Taxon Sphingomonadaceae, dem Stamm DSM 16255, zuordenbar dem Taxon Rhizobiales, dem Stamm DSM 16256, zuordenbar dem Taxon Microbacteriaceae, dem Stamm DSM 16253, zuordenbar dem Taxon Rhizobiaceae, dem Stamm DSM 16252, zuordenbar dem Taxon Alcaligenaceae und Pichia sp. DSM 16562, zur Detoxifizierung von Fumonisinen und Fumonisin-Derivaten in Nahrungsmitteln und/oder Futtermitteln. Durch die Verwendung der Mikroorganismen gemäß der vorliegenden Erfindung gelingt nicht nur eine vollständige Detoxifizierung von Fumonisinen und Fumonisin-Derivaten in Nahrungs- und/oder Futtermitteln, sondern neben der Tatsache, dass die Mikroorganismen zur Detoxifizierung in Medien fähig sind, die ein überschüssiges Kohlenstoffangebot zur Verfügung stellen, können diese Mikroorganismen die Detoxifizierung in extrem kurzer Zeit durchführen. Darüber hinaus gelingt es durch Verwendung der obengenannten Mikroorganismen, neben Fumonisinen wenigstens ein weiteres Mykotoxin, gewählt aus Zearalenonen, Aflatoxinen oder Ochratoxinen, zu detoxifizieren. Durch eine derartige Verwendung kann insbesondere in Mischfuttermitteln bzw. gemischten Getreideprodukten für den menschlichen Verzehr sichergestellt werden, dass mehrere im Getreide vorhandene Toxine durch Einsatz der erfindungsgemäßen Mikroorganismen sicher und rasch abgebaut werden.

Um diesen Abbau weiter zu vervollständigen, werden gemäß der Erfindung Mischkulturen aus Bakterien und/oder Hefen zur Detoxifizierung der Mykotoxine eingesetzt. Durch die Verwendung von Mischkulturen ist ein gezielter Angriff auf eine Mehrzahl von vorhandenen Toxinen gleichzeitig auf ein und demselben Nahrungs- bzw. Futtermittel bzw. Futtermittelgemisch möglich und somit eine vollständige Entgiftung derselben erzielbar. Des Weiteren wird es durch eine derartige Verwendung erstmals möglich, das Auftreten unerwünschter synergistischer Effekte infolge des gleichzeitigen Vorkommens mehrerer Mykotoxinarten sicher zu verhindern bzw. zu unterbinden.

Durch Verwendung der Mikroorganismen gemäß der Erfindung gelingt es darüber hinaus, extrem niedrige Konzentrationen der unterschiedlichsten Mykotoxine abzubauen, insbesondere von 100 µg/kg bis 500 mg/kg, vorzugsweise 250 µg/kg bis 25 mg/kg, Fumonisinen und Fumonisin-Derivaten, 10 µg/kg bis 10 mg/kg, vorzugsweise 40 µg/kg bis 2 mg/kg, Zearalenon bzw. Zearalenon-Derivate, 1 µg/kg bis 2 mg/kg, vorzugsweise 10 µg/kg bis 750 µg/kg, Aflatoxinen 1 µg/kg bis 2 mg/kg, vorzugsweise 5 µg/kg bis 500 µg/kg Ochratoxinen, wodurch es neben der Tatsache, dass eine Entgiftung von unterschiedlichsten Toxinen durch die Verwendung der erfindungsgemäßen Mikroorganismen möglich ist, auch sichergestellt wird, dass auch extrem niedrige Konzentrationen dieser Toxine angegriffen werden und angebaut werden, was mit herkömmlichen Mikroorganismen bis dato schwer, wenn nicht sogar unmöglich war.

Um eine möglichst vollständige Detoxifizierung sämtlicher, beispielsweise in einem Mischfuttermittel enthaltenen Toxine zu erzielen, ist die erfindungsgemäße Verwendung dahingehend weitergebildet, dass eine Kombination bzw. Mischkultur, enthaltend zusätzlich wenigstens ein weiteres Bakterium oder eine Hefe, gewählt aus: Sphingomonas sp. DSM 14170 und DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zum Detoxifizieren von Mykotoxinen, insbesondere Fumonisinen und Fumonisin-Derivaten, Zearalenon bzw. Zearalenon-Derivaten, Ochratoxinen, Trichothecenen und/oder Aflatoxinen, eingesetzt wird. Durch Verwendung einer Kombination bzw. Mischkultur, die zusätzlich wenigstens ein weiteres Bakterium oder eine Hefe enthält, welche(s) insbesondere zum Abbau von Trichothecenen, Zearalenon bzw. Zearalenon-Derivate, Aflatoxinen oder Ochratoxinen geeignet ist (sind), gelingt es, neben der detoxifizierenden Wirkung der Mikroorganismen gemäß der vorliegenden Erfindung, nämlich dem Abbau von Fumonisinen, deren Abbaufähigkeit hinsichtlich anderer Toxine dahingehend zu erweitern, dass durch eine gezielte Verwendung mehrerer Mikroorganismen sämtliche, in einem Futtermittel möglicherweise vorhandenen Toxine gemeinsam und unabhängig voneinander rasch und vollständig abgebaut werden können.

Bei einem Verfahren zur Entgiftung von Fumonisinen und Fumonisin-Derivaten unter Verwendung eines Mikroorganismus gemäß der vorliegenden Erfindung wird im wesentlichen so vorgegangen, dass Fumonisine im Futter mit bestimmten Keimzahlen enzymatisch in einer mehrstufigen Reaktion in einem deaminierten Metaboliten abgebaut werden. Gemäß einer Weiterbildung wird bevorzugt die Detoxifizierung unter wässrigen Bedingungen in Minimalmedium oder komplexen Umgebungen mit überschüssiger Nahrungszufuhr und Kohlenstoffquellen durchgeführt. Eine derartige Verfahrensführung erlaubt es, die Mikroorganismen gemäß der vorliegenden Erfindung in Verfahren einzusetzen, in welchen die Detoxifizierung direkt im Futtermittel durchgeführt wird, ohne Berücksichtigung der Menge an Kohlenstoff, die den Mikroorganismen zur Verfügung gestellt wird. Dies ist insbesondere dahingehend wichtig, da der größte Teil der bis dato bekannten Mikroorganismen lediglich in der Lage ist, ihre detoxifizierende Wirkung in Minimalmedium bzw. in Umgebungen mit nicht erhöhter Kohlenstoff zufuhr zu zeigen, was die meisten bekannten Mikroorganismen für einen direkten Einsatz in Nahrungs- und Futtermitteln aufgrund des übermäßigen Kohlenstoffangebots ungeeignet macht.

Gemäß einer bevorzugten Weiterbildung wird das Verfahren so geführt, dass es innerhalb von 15 min bis 12 h, insbesondere 15 min bis 2 h, durchgeführt wird. Bei einer derartigen Verfahrensführung kann einerseits sichergestellt werden, dass sämtliche, im Nahrungs- bzw. Futtermittel vorhandenen Mykotoxine, insbesondere Fumonisine, abgebaut sind und andererseits gelingt es dadurch nicht nur, die Mykotoxine abzubauen, sondern auch diesen Abbau in so rascher Zeit durchzuführen, dass ein derartiges Verfahren im großtechnischen Einsatz und nicht nur im Labormaßstab zur Anwendung kommen kann.

Wenn, wie dies einer Weiterbildung des Verfahrens gemäß der vorliegenden Erfindung entspricht, eine Kombination bzw. Mischkultur, enthaltend zusätzlich wenigstens ein weiteres Bakterium oder eine Hefe, gewählt aus Sphingomonas sp. DSM 14170 und DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zur Entgiftung von Mykotoxinen, insbesondere Fumonisinen und Fumonisin-Detivaten, Zearalenon bzw. Zearalenon-Derivate, Ochratoxinen, Trichothecenen und/oder Aflatoxinen, eingesetzt wird, wird neben dem Abbau von Fumonisinen bzw. Fumonisin-Derivaten und dem Abbau der Mykotoxine, welche durch die Mikroorganismen gemäß der vorliegenden Erfindung zusätzlich abgebaut werden können, das Verfahren so geführt, das ein vollständige Entgiftung von Nahrungs-und/oder Futtermitteln durch Einsatz einer gezielten Auswahl von Mikroorganismen erzielt wird.

Um die Entgiftung mit Sicherheit vollständig durchzuführen, wird, wie dies einer Weiterbildung der Erfindung entspricht, zur Entgiftung von Nahrungsmitteln und/oder Futtermitteln die Mikroorganismen mit den Nahrungs- und/oder Futtermitteln jeweils in einer Menge von 0,01 Gew.-% bis 1,5 Gew.-%, insbesondere 0,05 Gew.-% bis 0,7 Gew.-%, vermischt.

Die Erfindung umfasst schließlich einen Futtermittelzusatz zur Inaktivierung von Mykotoxinen, insbesondere Fumonisinen und Fumonisin-Derivaten, welcher dadurch gekennzeichnet ist, dass der Futtermittelzusatz einen Mikroorganismus nach einem der Ansprüche 1 bis 4 in einer Keimzahl von 2x10⁸/kg Futtermittelzusatz bis 2x10¹⁵/kg Futtermittelzusatz, insbesondere 1x10⁹/kg Futtermittelzusatz bis 5x10¹²/kg Futtermittelzusatz enthält. Durch Einsatz von Futtermittelzusätzen, die die Mikroorganismen mit Keimzahlen von 2x10⁸/kg Futtermittelzusatz bis 2x10¹⁵/kg Futtermittelzusatz enthalten, wird sichergestellt, dass eine vollständige Entgiftung von sämtlichen, durch die Mikroorganismen gemäß der vorliegenden Erfindung abbaubaren Fumonisinen und Fumonisin-Derivaten bewirkt wird und darüber hinaus auch sämtlich weiteren Toxine, die durch die Mikroorganismen gemäß der Erfindung abbaubar sind, abgebaut werden können.

Um diesen Abbau auch auf Mykotoxine auszudehnen, welche durch die Mikroorganismen gemäß der Erfindung nur teilsweise bzw. unvollständig abgebaut werden können, ist der Futtermittelzusatz dahingehend weitergebildet, dass er zusätzlich wenigstens ein weiteres Bakterium oder eine Hefe, gewählt aus Sphingomonas sp. DSM 14170 und DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zur Entgiftung von Mykotoxinen, insbesondere Fumonisinen und Fumonisin-Derivaten, Zearalenon bzw. Zearalenon-Derivate, Ochratoxinen, Trichothecenen und/oder. Aflatoxinen, enthält. Durch eine gezielte Kombination von mehreren Mikroorganismen gelingt somit ein vollständiger Abbau sämtlicher Toxine in ein und demselben Futtermittel, wodurch insbesondere die synergistische Wirkung von mehreren Toxinen in einem Nahrungs- bzw. Futtermittel mit Sicherheit vermieden werden kann.

Futtermittelzusätse gemäß der vorliegenden Erfindung sind, wie dies einer weiterbildung der Erfindung entspricht, zur Inaktivierung von Fumonisin B1, B2, B3 und Fumonisin-Derivaten, Zearalenon Zearalenol, Zearalenon-Glykosiden, Aflatoxin B1, B2, G1, G2, M1, M1, Deoxynivalenol (DON), T-2 Toxin, HT-2 Toxin, Nivalenol, Monoacetoxyscirpenol, Diacetoxyscirpenol, Triehodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol und culmorin und/oder Ochratoxin A, B, C, D in einem Futtermittel von Tieren geeignet.

Die Erfindung wird nachfolgend anhand von Beispielen näher erläutert, in welchen in Beispiel 1 der zeitliche Verlauf des Abbaus von Fumonisin B1 bei konstanter Toxinkonzentration in Minimalmedium gezeigt ist, in Beispiel 2 der Abbau von Fumonisin B1 bei unterschiedlichen Toxinkonzentrationen gezeigt ist, in Beispiel 3 der Abbau von Fumonisin B1 in komplexen Medien gezeigt ist, in Beispiel 4 der Abbau von Fumonisin B1 in Nahrungs- und Futtermitteln, in Beispiel 5 der Abbau von Ochratoxin mit den Mikroorganismen gemäß der Erfindung gezeigt ist und in Beispiel 5 Fütterungsversuche unter Einsatz einer Mikroorganismenmischung gemäß der vorliegenden Erfindung gezeigt ist.

### Beispiel 1

Abbau bzw. Detoxifizierung von Fumonisin B1 in Minimalmedium bei einer Toxinkonzentration von 2 mg/l Fumonisin B1

Für die Versuche wurden die Mikroorganismen DSM 16254 und DSM 16257 sowie als Vergleich der Stamm Exophiala spinifera DSM 1217 eingesetzt.

Die Inkubation erfolgte in allen Fällen bei 25 °C unter aeroben Bedingungen. Die Anzucht der Mikroorganismen erfolgte unter Anwesenheit von 50 mg/l Fumonisin B1 im allgemeinen Anzuchtmedium, um eine eventuell mögliche Induktion der Fumonisin B1 detoxifizierenden Enzyme zu ermöglichen.

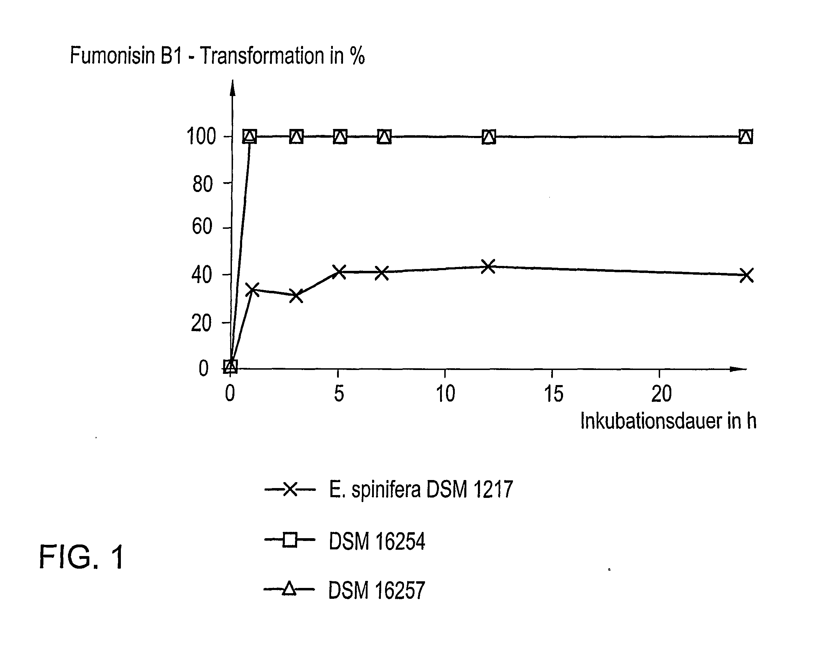

Aus Fig. 1 ist ersichtlich, daß die Stämme DSM 16254 und DSM 16257 Fumonisin B1 bereits nach 1 h Inkubation zu 100 % transformiert haben, während der Vergleichs-Hefestamm E. spinifera nach 24 h Inkubationsdauer lediglich knapp 41 % des Toxins transformieren konnte. Die Mikroorganismen gemäß der vorliegenden Erfindung sind somit nicht nur in der Lage extrem rasch in Minimalmedium Fumonisin B1 zu detoxifizieren, sondern diese Detoxifizierung findet zu 100 % statt.

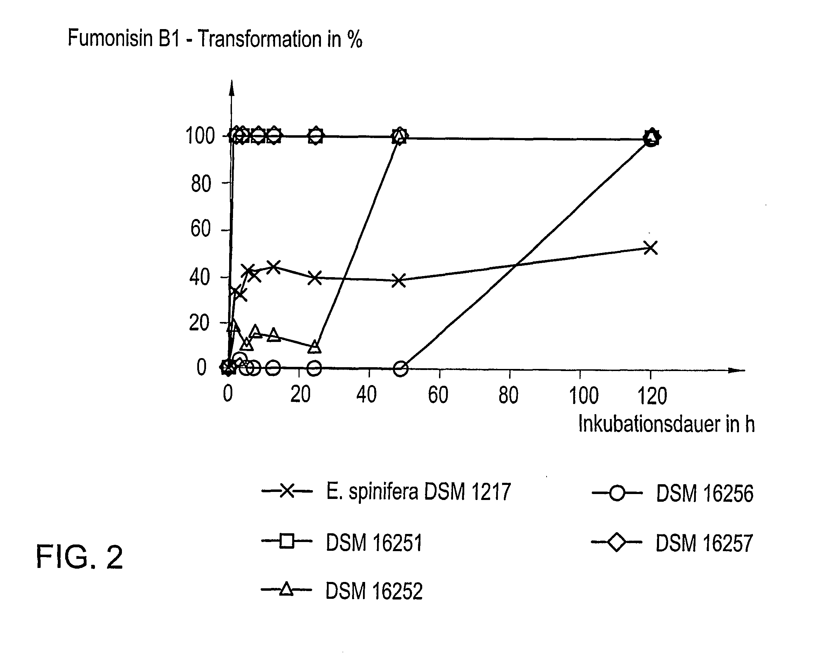

Fig. 2 zeigt die Transformation im zeitlichen Verlauf im selben Versuchsansatz, d.h. Minimalmedium und Toxinkonzentration von 2 mg/l, für die Stämme DSM 16254, DSM 16256, DSM 16252, DSM 16257 sowie den Hefestamm E. spinifera DSM 1217 als Vergleich. Aus diesen Abbauversuchen ergibt sich deutlich, daß die Mikroorganismen gemäß der Erfindung extrem rasch und in vielen Fällen, nämlich DSM 16254, DSM 16257, DSM 16252, DSM 16256, auch zu 100 % abgebaut werden, was mit den Vergleichs-Mikroorganismus, DSM 1217, nicht möglich war.

### Beispiel 2

Abbau von Fumonisin B1 bei unterschiedlichen Toxinkonzentrationen

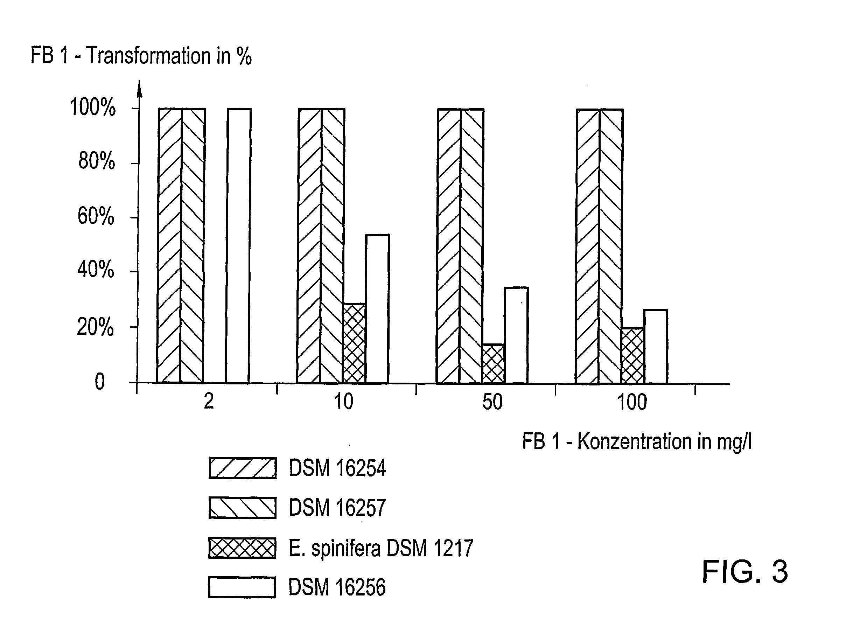

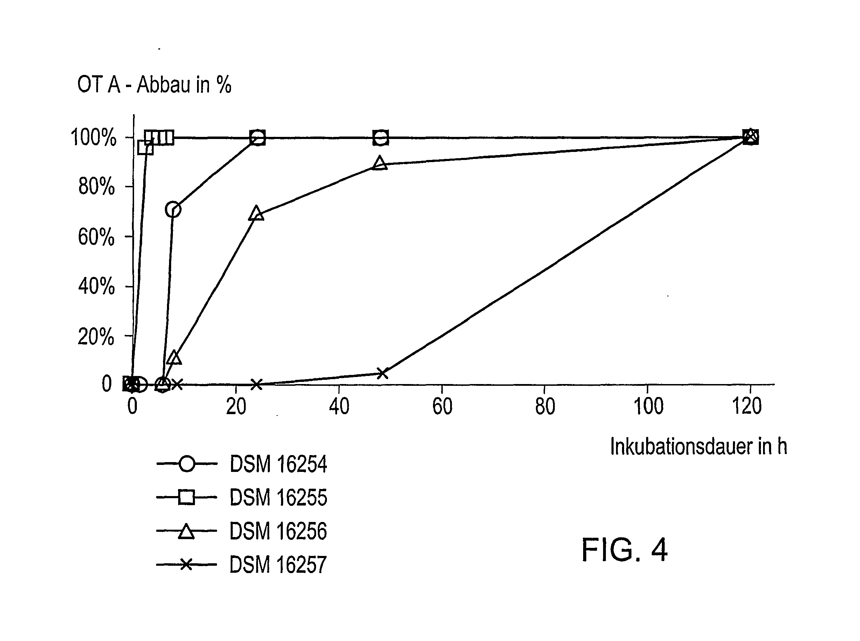

Die Versuche wurden mit DSM 16254, DSM 16257 und DSM 16256 durchgeführt sowie als Vergleich mit dem Hefestamm Exophiala spinifera DSM 1217. Die eingesetzten Toxinkonzentrationen waren 2, 10, 50, 100 und 500 mg/l Fumonisin B1. Die Inkubation erfolgte unter aeroben Bedingungen bei 25 °C. Die Ergebnisse sind nach 5 h Inkubation der Ansätze dargestellt, da derartige Inkubationsdauern ein praxisrelevanter Zeitraum hinsichtlich einer Detoxifikation von Fumonisinen in Futtermitteln sind. Fig. 3 zeigt die Ergebnisse dieses Versuchs. Der Mikroorganismus DSM 16254 konnte in sämtlichen Konzentrationsbereichen Fumonisin B1 zu 100 % abbauen, der Mikroorganismus DSM 16257 konnte lediglich im Konzentrationsbereich von 100 mg/l Fumonisin B1 einen 96 %-igen Abbau erzielen, DSM 16256 ermöglichte einen 100 %-igen Abbau bei einer Konzentration von 2 mg/l, einen über 50 %-igen Abbau in der Konzentration von 10 mg/l, einen 35 bzw. 25 %-igen Abbau bei den Konzentrationen von 50 mg/l bzw. 100 mg/l. Der Vergleich mit E. spinifera DSM 1217 zeigte, daß bei diesem Mikroorganismus insbesondere bei extrem niedrigen Toxinkonzentrationen ein extrem schlechtes Abbauvermögen bestand, bei 10 mg/l zeigte DSM 1217 die beste Aktivität und baute Fumonisin B1 zu etwa 30 % ab. Aus diesem Vergleich ergibt sich, daß die Mikroorganismen gemäß der vorliegenden Erfindung DSM 1217 in sämtlichen Konzentrationsbereichen überlegen sind und daß insbesondere bei niedrigen Toxinkonzentrationen ein 100 %-iger Abbau möglich ist, was mit Mikroorganismen gemäß dem Stand der Technik bis dato nicht möglich war.

### Beispiel 3

### Abbau von Fumonisin B1 in komplexem Medium

Bei diesem Versuch wurde die Fähigkeit der Mikroorganismen untersucht, ob sie Fumonisin B1 auch in komplexen Medien bei Anwesenheit hoher Nährstoffkonzentrationen detoxifizieren können, untersucht. Die Anzucht der Mikroorganismen erfolgte in einem komplexen Nährmedium bestehend aus 5g/l Pepton aus Fleischextrakt und 3 g/l Fleischextrakt, welches mit zwei unterschiedlichen Konzentrationen von Fumonisin B1, nämlich 10 mg/l und 100 mg/l, versetzt wurde. Die Bestimmung der Transformationsraten erfolgte durch Vergleich der Toxingehalte in den Ansätzen zu Beginn und am Ende einer 72-stündigen Inkubation bei 25 °C unter aeroben Bedingungen. In beiden Fällen gelang eine 100 %-ige Detoxifizierung bzw. ein 100 %-iger Abbau von Fumonisin B1 bei Anwesenheit von 10 mg/l Fumonisin B1 im Medium. Auch bei Anwesenheit von 100 mg/l Fumonisin B1 gelang in beiden Fällen eine 100 %-ige Detoxifizierung. Mit diesem Versuch konnte eindeutig bewiesen werden, daß die Mikroorganismen gemäß der Erfindung zum Abbau von Fumonisinen in komplexen Medien, d.h. mit erhöhtem Nährstoffangebot, geeignet sind.

### Beispiel 4

### Abbau von Fumonisin B1 in Nahrungs- und Futtermitteln

Es wurden wiederum die Mikroorganismen DSM 16254 bzw. DSM 16257 herangezogen und es wurde versucht, eine Toxinkonzentration von 10 mg/l Fumonisin B1 in Bier, Maisgrieß und Weizengrieß abzubauen. Nach Anzucht der Mikroorganismen wurden diese geerntet, in toxinhaltiger Pufferlösung resuspendiert und anschließend sofort mit dem entsprechenden Lebens- bzw. Futtermittel inkubiert. Die Abbaurate von Fumonisin B1 betrug in allen Fällen 100 %, so daß eindeutig nachgewiesen werden konnte, daß die Mikroorganismen gemäß der Erfindung in Futtermitteln bzw. Nahrungsmitteln Fumonisine zu 100 % abbauen können.

### Beispiel 5

### Abbau von anderen Mykotoxinen durch die Mikroorganismen gemäß der Erfindung

Als beispielhaftes Mykotoxin wurde im vorliegenden Fall Ochratoxin A herangezogen. Es gelangten die Stämme DSM 16254, DSM 16255, DSM 16256 und DSM 16257 zum Einsatz. Der Ochratoxin-Abbau wurde in Gegenwart von 400 µg/l Ochratoxin A in Aerobpuffer durchgeführt und es wurde 120 h inkubiert. Der Stamm DSM 16255 zeigte bereits nach 2 h eine 95 %-ige Detoxifizierung, nach 24 h hatten sowohl der Stamm DSM 16254 als auch DSM 16255 100 % des Ochratoxins A detoxifiziert, nach 48 h konnte auch bei DSM 16256 bereits eine 90 %-ige Detoxifizierung festgestellt werden und nach 120 h hatte auch der Stamm DSM 16257 Ochratoxin A zu 100 % detoxifiziert.

### Beispiel 6

### Fütterungsversuche mit Kombinationen bzw. Mischkulturen aus verschiedenen Mikroorganismen zur vollständigen Detoxifizierung von mit Mykotoxinen versetzten Nahrungs- bzw. Futtermitteln

### Ferkelversuch I

In diesem Versuch wurden die Stämme DSM 16254 und DSM 14153 als Additiv eingesetzt. Das Additiv hatte eine Gesamtkeimzahl von 1x10¹²KBE/kg Additiv. Die Versuchsdauer betrug 42 Tage. Die Tiere wurden in vier Gruppen zu je 24 Tiere eingeteilt. Die Kontrollgruppe (KG) erhielt unkontaminiertes Standardfutter ohne Futterzusatz. Die Toxingruppe (TG) erhielt ein Futter, das mit 500 ppb Ochratoxin A, 250 ppb Zearalenon und 1.500 ppb Fumonisin B1 versetzt war. Die Versuchsgruppe 1 (VG1) und die Versuchsgruppe 2 (VG2) erhielten jeweils dasselbe mit Toxinen versetzte Futter, im Fall der Versuchsgruppe 1 jedoch mit 0,5 kg Additiv und im Fall der Versuchsgruppe 2 1 kg Additiv. Am Ende des Versuchs konnten folgende Ergebnisse erzielt werden.

| | Gewicht am Ende | tägliche Gewichtszunahmen | FCR |
|---|---|---|---|
| KG | 24,3 kg | 434 g | 1,493 |
| TG | 22,0 kg | 380 g | 1,573 |
| VG1 | 23,4 kg | 412 g | 1,516 |
| VG2 | 24,7 kg | 443 g | 1,467 |

### Ferkelversuch II

In diesem Versuch wurde DSM 16254, DSM 11798 und DSM 14153 als Additiv eingesetzt. Das Additiv hatte eine Gesamtkeimzahl von 2,5x10¹²KBE/kg Additiv. Die Versuchsdauer betrug 42 Tage. Die Tiere wurden in vier Gruppen zu je 19 Tiere eingeteilt. Die Toxingruppe (TG) erhielt ein Futter, das mit 1,1 ppm Deoxynivalenol und 2 ppm Fumonisin B1 versetzt war, jedoch kein Additiv. Die Versuchsgruppe 1 (VG1), die Versuchsgruppe 2 (VG2) und die Versuchsgruppe 3 (VG3) erhielten jeweils dasselbe mit Toxinen versetzte Futter, im Fall der Versuchsgruppe 1 jedoch mit 0,5 kg Additiv, im Fall der Versuchsgruppe 2 1 kg Additiv und im Fall der Versuchsgruppe 3 2 kg Additiv. Am Ende des Versuchs konnten folgende Ergebnisse erzielt werden.

| | Gewicht am Ende | tägliche Gewichtszunahmen | FCR |
|---|---|---|---|
| TG | 22,90 kg | 359 g | 1,82 |
| VG1 | 26,45 kg | 442 g | 1,67 |
| VG2 | 27,10 kg | 463 g | 1,60 |
| VG3 | 28,55 kg | 485 g | 1,71 |

### Ferkelversuch III

In diesem Versuch wurden die Stämme DSM 16254 als Additiv eingesetzt. Das Additiv hatte eine Gesamtkeimzahl von 1x10¹¹KBE/kg Additiv. Die Versuchsdauer betrug 42 Tage. Die Tiere wurden in zwei Gruppen zu je 30 Tiere eingeteilt. Die Toxingruppe (TG) erhielt ein Futter, das mit 4,5 ppm Fumonisin B1 versetzt war. Die Versuchsgruppe erhielt dasselbe mit Toxinen versetzte Futter, jedoch mit 0,5 kg Additiv. Am Ende des Versuchs konnten folgende Ergebnisse erzielt werden.

| | Anfangsgewichte | Endgewichte | Tageszunahmen | Futterverwertungsrate |
|---|---|---|---|---|
| Toxingruppe | 6,76 kg | 28,33 kg | 514 g | 2,21 |
| Versuchsgruppe | 6,88 kg | 30,56 kg | 564 g | 2,03 |

### Broiler-Versuch I

In diesem Versuch wurden die Stämme DSM 16254 als Additiv eingesetzt. Das Additiv hatte eine Gesamtkeimzahl von 2,5x10¹¹ KBE/kg Additiv. Die Tiere wurden in zwei Gruppen zu je 140.000 Tiere eingeteilt. Die Toxingruppe (TG) erhielt ein Futter, das mit 300 ppb Aflatoxin und 2 ppm Fumonisin versetzt war. Die Versuchsgruppe erhielt dasselbe mit Toxinen versetzte Futter, jedoch mit 1 kg Additiv/t Futtermittel. Am Ende des Versuchs konnten folgende Ergebnisse erzielt werden.

| Versuchs-Woche | Versuchsgruppe | Toxingruppe |
|---|---|---|
| | Mortalität [%] | Mortalität [%] |
| 1 | 1,02 | 2,99 |
| 2 | 0,82 | 1,83 |
| 3 | 0,34 | 1,79 |
| 4 | 0,57 | 2,89 |
| 5 | 0,89 | 2,27 |
| 6 | 0,91 | 1,24 |
| 7 | 0,73 | 1,07 |
| 8 | 0,41 | 1,63 |
| End-Gewicht [g] | 1720 | 1298 |

### Broiler-Versuch II

In diesem Versuch wurden die Stämme DSM 16254 und DSM 11798 als Additiv eingesetzt. Das Additiv hatte eine Gesamtkeimzahl von 4x10¹¹KBE/kg Additiv. Die Tiere wurden in drei Gruppen zu je 260 Tiere eingeteilt. Die Kontrollgruppe erhielt unkontaminiertes Futter. Die Toxingruppe (TG) erhielt ein Futter, das mit 3,5 ppm Fumonisin und 1,8 ppm T-2-Toxin versetzt war. Die Versuchsgruppe erhielt dasselbe mit Toxinen versetzte Futter, jedoch mit 1kg Additiv pro Tonne Futter. Am Ende des Versuchs konnten folgende Ergebnisse erzielt werden.

| | Kontrollgruppe | Versuchsgruppe | Toxingruppe |
|---|---|---|---|
| Gesamt-Gewichtszunahme | 1965,6 | 1952,4 | 1866,1 |
| Gesamt-Futteraufnahme | 3983,9 | 4113,2 | 3894,0 |
| Futterverwertungs-Rate | 2,03 | 2,11 | 2,08 |

## Patentansprüche

1. Mikroorganismus zur Entgiftung von Fumonisinen und Fumonisin-Derivaten, wobei detoxifizierende Bakterien oder Hefen, gewählt au den Stämmen DSM 16254 und DSM 16257, zuordenbar dem Taxo Sphingomonadaceae, dem Stamm DSM 16255, zuordenbar dem Taxon Rhizobiales, dem Stamm DSM 16256, zuordenbar dem Taxon Microbacteriaceae, dem Stamm DSM 16253, zuordenbar dem Taxon Rhizobiaceae, dem Stamm DSM 16252, zuordenbar dem Taxon Alcaligenaceae und Pichia sp. DSM 16562, eingesetzt sind, welche Fumonisine enzymatisch in einer mehrstufigen Reaktion in deaminierte Metaboliten umwandeln.

2. Mikroorganismus nach Anspruch 1, **dadurch gekennzeichnet, dass** die Bakterien oder Hefen in Form von Pulver, Flüssigkeiten oder Gel stabilisiert sind.

3. Mikroorganismus nach Anspruch 1 oder 2, **dadurch gekennzeichnet, dass** die Bakterien oder Hefen neben Fumonisin und Fumonisin-Derivaten wenigstens ein weiteres Mykotoxin, gewählt aus Zearalenonen, Aflatoxinen oder Ochratoxinen, detoxifizieren.

4. Verwendung von Bakterien oder Hefen, alleine oder in Kombination von zwei oder mehreren Stämmen, gewählt aus dem Stamm DSM 16254 und DSM 16257, zuordenbar dem Taxon Sphingomonadaceae, dem Stamm DSM 16255, zuordenbar dem Taxon Rhizobiales, dem Stamm DSM 16256, zuordenbar dem Taxon Microbacteriacea, dem Stamm DSM 16253, zuordenbar dem Taxon Rhizobiaceae, dem Stamm DSM 16252, zuordenbar dem Taxon Alcaligenaceae und Pichia sp. DSM 16562, zur Detoxifizierung von Fumonisinen und Fumonisin-Derivaten in Nahrungsmitteln und/oder Futtermitteln.

5. Verwendung nach Anspruch 4, **dadurch gekennzeichnet, dass** die Bakterien oder Hefen zusätzlich zur Detoxifizierung von wenigstens einem weiteren Mykotoxin, gewählt aus Zearalenonen, Aflatoxinen oder Ochratoxinen, eingesetzt werden.

6. Verwendung nach Anspruch 4 oder 5, **dadurch gekennzeichnet, dass** Mischkulturen aus Bakterien und/oder Hefen zur Detoxifizierung der Mykotoxine eingesetzt werden.

7. Verwendung nach Anspruch 4, 5 oder 6 zum Entgiften von niedrigen Mykotoxin-Konzentrationen, insbesondere von 100 µg/kg bis 500 mg/kg, vorzugsweise 250 µg/kg bis 25 mg/kg, Fumonisinen und Fumonisin-Derivaten, 10 µg/kg bis 10 mg/kg, vorzugsweise 40 µg/kg bis 2 mg/kg, Zearalenon bzw. Zearalenon-Derivate, 1 µg/kg bis 2 mg/kg, vorzugsweise 10 µg/kg bis 750 µg/kg, Aflatoxinen, 1 µg/kg bis 2 mg/kg, vorzugsweise 5 µg/kg bis 500 µg/kg, Ochratoxinen.

8. Verwendung nach einem der Ansprüche 4 bis 7, **dadurch gekennzeichnet, dass** eine Kombination bzw. Mischkultur, enthaltend zusätzlich wenigstens ein weiteres Bakterium oder eine Hefe, gewählt aus: Sphingomonas sp. DSM 14170 und DSM 14167, Stenotropomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zum Detoxifizieren von Mykotoxinen, insbesondere Fumonisinen und Fumonisin-Derivaten, Zearalenon bzw. Zearalenon-Derivate, Ochratoxinen, Trichothecenen und/oder Aflatoxinen, zum Detoxifizieren von gemeinsam auftretenden Mykotoxinen, insbesondere Fumonisin und Fumonisin-Derivaten, Zearalenon bzw. Zearalenon-Derivate, Aflatoxinen oder Ochratoxinen eingesetzt wird.

9. Verfahren zur Entgiftung von Fumonisinen und Fumonisin-Derivaten unter Verwendung eines Mikroorganismus nach einem der Ansprüche 1 bis 4, umfassend enzymatisches Umwandeln von Fumonisinen im Futter mit einer Keimzahl von 10³/g Futter bis 10⁸/g Futter, insbesondere 2x10⁴/g Futter bis 5x10⁶/g Futter in einer mehrstufigen Reaktion in einen deaminierten Metaboliten.

10. Verfahren nach Anspruch 9, **dadurch gekennzeichnet, dass** das Entgiften unter wässrigen Bedingungen in Minimalmedium oder in komplexen Umgebungen mit überschüssiger Nahrungszufuhr und Kohlenstöffquellen durchgeführt wird.

11. Verfahren nach Anspruch 9 oder 10, **dadurch gekennzeichnet, dass** es innerhalb von 15 min bis 12 h, insbesondere 15 min bis 2 h, durchgeführt wird.

12. Verfahren nach Anspruch 9, 10 oder 11, **dadurch gekennzeichnet, dass** wenigstens ein weiteres Mykotoxin, gewählt aus Zearalenonen Aflatoxinen oder Ochratoxinen, in ein nicht-giftiges Abbauprodukt umgewandelt wird.

13. Verfahren nach einem der Ansprüche 9 bis 12, **dadurch gekennzeichnet, dass** eine Kombination bzw. Mischkultur, enthaltend zusätzlich wenigstens ein weiteres Bakterium oder eine Hefe, gewählt aus Sphingomonas sp. DSM 14170 und DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zur Entgiftung von Mykotoxinen, insbesondere Fumonisinen und Fumonisin-Derivaten, Zearalenon bzw. Zearalenon-Derivaten, Ochratoxinen, Trichothecenen und/oder Aflatoxinen, eingesetzt wird.

14. Verfahren nach einem der Ansprüche 9 bis 12, **dadurch gekennzeichnet, dass** zur Entgiftung von Nahrungsmitteln und/oder Futtermitteln die Mikroorganismen mit den Nahrungs- und/oder Futtermitteln jeweils in einer Menge von 0,01 Gew.-% bis 1,5 Gew.-%, insbesondere 0,05 Gew.-% bis 0,7 Gew.-%, vermischt werden.

15. Futtermittelzusatz zur Inaktivierung von Mykotoxinen, insbesondere Fumonisinen und Fumonisin-Derivaten, **dadurch gekennzeichnet, dass** der Futtermittelzusatz einen Mikroorganismus nach einem der Ansprüche 1 bis 4 in einer Keimzahl von 2x10⁸/kg Futtermittelzusatz bis 2x10¹⁵/kg Futtermittelzusatz, insbesondere 1x10⁹/kg Futtermittelzusatz bis 5x10¹²//kg Futtermittelzusatz enthält.

16. Futtermittelzusatz nach Anspruch 15, **dadurch gekennzeichnet, dass** er zusätzlich wenigstens ein weiteres Bakterium oder eine Hefe, gewählt aus Sphingomonas sp. DSM 14170 und DSM 14167, Stenotrophomonas, nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 oder Eubacterium DSM 11798, zur Entgiftung von Mykotoxinen, insbesondere Fumonisinen und Fumonisin-Derivaten, Zearalenon bzw. Zearalenon-Derivaten, Ochratoxinen, Trichothecenen und/oder Aflatoxinen, enthält.

17. Verwendung eines Futtermittelzusatzes nach Anspruch 15 oder 16 zur Inaktivierung von Fumonisin B1, B2, B3 und Fumonisin-Derivaten Zearalenon bzw. Zearalenon-Derivaten, Zearalenol, Zearalenon-Gylkoside, Aflatoxin B1, B2, G1, G2, M1, M1, Deoxynivalenol (DON), T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol und Culmorin und/oder Ochratoxin A, B, C, D.

## Claims

1. A microorganism for decontaminating fumosins and fumonisin derivatives, wherein detoxifying bacteria or yeasts selected from the strains DSM 16254 and DSM 15257, assignable to the taxon Sphingomonadaceae, strain DSM 16255, assignable to the taxon Rhizobiales, strain DSM 16256, assignable to the taxon Microbacteriaceae, strain DSM 16253, assignable to the taxon Rhizobiaceae, strain DSM 16252 assignable to the taxon Alcaligenaceae, and Pichia sp. DSM 16562, are used, which convert fumonisins enzymatically into deaminated metabolites in a multi-step reaction.

2. A microorganism according to claim 1, **characterized in that** said bacteria or yeasts are stabilized in the form of powders, liquids or gels.

3. A microorganism according to claim 1 or 2 **characterized in that** said bacteria or yeasts detoxify, in addition to fumonisin derivatives, at least one further mycotoxin from zearalenones, aflatoxins or ochratoxins.

4. The use of bacteria or yeasts, alone or in combination of two or more strains, selected from the strains DSM 16254 and DSM 15257, assignable to the taxon Sphingomonadaceae, strain DSM 16255, assignable to the taxon Rhizobiales, strain DSM 16256, assignable to the taxon Microbacteriaceae, strain DSM 16253, assignable to the taxon Rhizobiaceae, strain DSM 16252, assignable to the taxon Alcaligenaceae, and Pichia sp. DSM 16562, for detoxifying fumonisins und fumonisin derivatives in foods and/or feeds.

5. The use according to claim 4, **characterized in that** said bacteria or yeasts, in addition, are employed for detoxifying at least one further mycotoxin selected from zearalenones, aflatoxins or ochratoxins.

6. The use according to claim 4 or 5 **characterized in that** mixed cultures of bacteria and/or yeasts are used for detoxifying mycotoxins.

7. The use according to claim 4, 5 or 6, for decontaminating low mycotoxin concentrations and in particular, 100 µg/kg to 500 mg/kg, preferably 250 µg/kg to 25 mg/kg, fumonisins and fumonisin derivatives, 10 µg/kg to 10 mg/kg, preferably 40 µg/kg to 2 mg/kg, zearalenones and zearalenone derivatives, 1 µg/kg to 2 mg/kg, preferably 10 µg/kg to 750 µg/kg, aflatoxins, 1 µg/kg to 2 mg/kg, preferably 5 µg/kg to 500 µg/kg, ochratoxins.

8. The use according to any one of claims 4 to 7, **characterized in that** a combination or mixed culture additionally containing at least one further bacterium or yeast selected from Sphingomonas sp. DSM 14170 and DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 or Eubacterium DSM 11798 is used for detoxifying mycotoxins, in particular fumonisins and fumonisin derivatives, zearalenones and zearalenone derivatives, ochratoxins, trichothecenes and/or aflatoxins, for detoxifying, jointly occurring mycotoxins, in particular fumonisins and fumonisin derivatives, zearalenones and zearalenone derivatives, aflatoxins or ochratoxins.

9. A method for decontaminating fumonisins and fumonisin derivatives using a microorganism according to any one of claims 1 to 4, comprising enzymatically converting fumonisins in fodder with a germ count of from 10⁸/g fodder to 10³/g fodder, in particular 2x10⁴/g fodder to 5x10⁶/g fodder, into deaminated metabolites in a single-step or multi-step reaction.

10. A method according to claim 9, **characterized in that** said detoxification is carried out under aqueous conditions in minimal medium or complex environments with excess nutrient supply and carbon sources.

11. A method according to claim 9 or 10, **characterized in that** it is carried out within 15 min to 12 h and, in particular, 15 min to 2 h.

12. A method according to claim 9, 10 or 11, **characterized in that** at least one further mycotoxin selected from zearalenones, aflatoxins or ochratoxins is converted into a non-toxic degradation product.

13. A method according to any one of claims 9 to 12 **characterized in that** a combination or mixed culture additionally containing at least one further bacterium or yeast selected from Sphingomonas sp. DSM 14170 and DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 or Eubacterium DSM 11798 is used for detoxifying mycotoxins, in particular fumonisins and fumonisin derivatives, zearalenones and zearalenone derivatives, ochratoxins, trichothecenes and/or aflatoxins.

14. A method according to any one of claims 9 to 12 **characterized in that**, for the decontamination of foods and/or feeds, the microorganisms are mixed with said foods and/or feeds each in amounts ranging from 0.01% by weight to 1.5% by weight and, in particular, 0,05% by weight to 0.7% by weight.

15. A feed additive for inactivating mycotoxins, in particular fumonisins and fumonisin derivatives, **characterized in that** said feed additive contains a microorganism according to any one of claims 1 to 4 at a germ count of from 2x10⁸/kg feed additive to 2x10¹⁵/kg feed additive, in particular 1x10⁹/kg feed additive to 15x10¹²/kg feed additive.

16. A feed additive according to claim 15, **characterized in that** it additionally contains at least one further bacterium or yeast selected from Sphingomonas sp. DSM 14170 and DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162, or Eubacterium DSM 11798, for detoxifying mycotoxins, in particular fumonisins and fumonisin derivatives, zearalenones and zearalenone derivatives, ochratoxins, trichothecenes and/or aflatoxins.

17. The use of a feed additive according to claim 15 or 16 for inactivating fumonisins B1, B2, B3 and fumonisin derivatives, zearalenones and zearalenone derivatives, zearalenol, zearalenone glycosides, aflatoxins B1 B2, G1, G2, M1, M1, deoxynivalenol (DON), T-2 toxin, HT-2 toxin, nivalenol, monoacetoxyscirpenol, diacetoxyscirpenol, trichodermol, verrucarin, rorodin, acetyl deoxynivalenol, isotrichodermin, hydroxyisotrichodermin, calonectrin, T-2 tetraol, T-2 triol, deacetylneosolaniol, neosolaniol, acetylneosolaniol, sporotrichiol, trichotriol, sambucinol and culmorin and/or ochratoxins A, B, C, D.

## Revendications

1. Microorganisme pour détoxication des fumonisines et des dérivés de fumcnisine dans lequel on utilise des bactéries ou des levures détoxifiantes choisies parmi les souches DSM 16254 et DSM 16257, attribuables au taxon Sphingomonadaceae, la souche DSM 16255 attribuable au taxon Rhizobiales, la souche DSM 16256, attribuable au taxon Micobacteriaceae, la souche DSM 16253 attribuable au taxon Rhizobiaceae, la souche DSM 16252, attribuable au taxon alcaligenaceae et Pichia sp. DSM 16562, qui convertissent les fumonisines par voie enzymatique dans une réaction à plusieurs étapes en métabolites désaminés.

2. Microorganisme selon la revendication 1, **caractérisé en ce que** les bactéries ou les levures sont stabilisées sous forme de poudre, de fluides ou de gel.

3. Microorganisme selon la revendication 1 ou 2, **caractérisé en ce que** les bactéries ou les levures détoxifient, en plus de la fumonisine et aux dérivés de fumonisine, au moins une autre mycotoxine choisie parmi les zéaralénones, les aflatoxines ou les ochratoxines.

4. Utilisation de bactéries ou de levures, seules ou en combinaison de deux souches ou plus, choisies parmi les souches DSM 16254 et DSM 16257, attribuables au taxon Sphingomonadaceae, la souche DSM 16255 attribuable au taxon Rhizobiales, la souche DSM 16256, attribuable au taxon Micobacteriaceae, la souche DSM 16253 attribuable au taxon Rhizobiaceae, la souche DSM 16252, attribuable au taxon Alcaligenaceae et Pichia sp. DSM 16562 pour la détoxification de fumonisines et de dérivés de fumonisine dans des produits alimentaires et/ou des produits alimentaires pour animaux.

5. Utilisation selon la revendication 4, **caractérisée en ce que** les bactéries ou les levures sont utilisées en outre pour la détoxification d'au moins une autre mycotoxine choisie parmi les zéaralénones, les aflatoxines ou les ochratoxines.

6. Utilisation selon la revendication 4 ou 5, **caractérisée en ce que** les cultures mixtes de bactéries et/ou de levures sont utilisées pour la détoxification des mycotoxines.

7. Utilisation selon la revendication 4, 5 ou 6, pour la décontamination de concentrations de mycotoxine inférieures, en particulier de 100 µg/kg à 500 mg/kg, de préférence 250 µg/kg à 25 mg/kg, de fumonisines et de dérivés de fumonisine, de 10 µg/kg à 10 mg/kg, de préférence de 40 µg/kg à 2 mg/kg, de zéaralénone ou de dérivés de zéaralénone, de 1 µg/kg à 2 mg/kg, de préférence de 10 µg/kg à 750 µg/kg d'aflatoxines, de 1 µg/kg à 2 mg/kg, de préférence de 5 µg/kg à 500 µg/kg d'ochratoxines.

8. Utilisation selon l'une des revendications 4 à 7, **caractérisée en ce que** l'on utilise une combinaison ou une culture mixte contenant en outre au moins une autre bactérie ou une levure choisie parmi Sphingomonas sp. DSM 14170 et DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 ou Eubacterium DSM 11798 pour la détoxification de mycotoxines, en particulier de fumonisines et de dérivés de fumonisine, de zéaralénone ou de dérivés de zéaralénone, d'ochratoxines, de trichothécènes et/ou d'aflatoxines, pour la détoxification de mycotoxines apparaissant conjointement, en particulier, de la fumonisine et des dérivés de fumonisine, de zéaralénone ou de dérivés de zéaralénone, d'aflatoxines ou d'ochratoxines.

9. Procédé de décontamination de fumonisines et de dérivés de fumonisine en utilisant un microorganisme selon l'une des revendications 1 à 4, comprenant la conversion enzymatique de fumonisines dans l'aliment pour animaux, avec un nombre de germes de 10³/g d'aliment à 10⁸/g d'aliment, en particulier, 2 x 10⁴/ g d'aliment à 5 x 10⁶/g d'aliment dans une réaction en plusieurs étapes en un métabolite désaminé.

10. Procédé selon la revendication 9, **caractérisé en ce que** la décontamination est réalisée dans des conditions aqueuses en milieu minimal ou dans des environnements complexes avec un apport alimentaire excédentaire et des sources de carbone.

11. Procédé selon la revendication 9 ou 10, **caractérisé en ce qu'**il est réalisé en l'espace de 15 min à 12 h, en particulier, de 15 min à 2 h.

12. Procédé selon la revendication 9, 10 ou 11, **caractérisé en ce qu'**au moins une autre mycotoxine choisie parmi les zéaralénones, les aflatoxines ou les ochratoxines est convertie en un produit de décomposition non toxique.

13. Procédé selon l'une des revendications 9 à 12, **caractérisé en ce que** l'on utilise une combinaison ou une culture mixte contenant en outre au moins une autre bactérie ou une levure choisie parmi Sphingomonas sp. DSM 14170 et DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodotorula yarrowii DSM 14155, Trichosporon mucoides DSM 14156, Trichosporon dulcitum DSM 14162 ou Eubacterium DSM 11798 pour la détoxification de mycotoxines, en particulier de fumonisines et de dérivés de fumonisine, de zéaralénone ou de dérivés de zéaralénone, d'ochratoxines, de trichothécènes et/ou d'aflatoxines.

14. Procédé selon l'une des revendications 9 à 12, **caractérisé en ce que**, pour la décontamination des produits alimentaires et/ou des aliments pour animaux, les microorganismes sont mélangés avec les produits alimentaires et/ou produits alimentaires pour animaux, respectivement en une quantité de 0,01 % en poids à 1,5 % en poids, en particulier de 0,05 % en poids à 0,7 % en poids.

15. Additif d'aliment pour animaux pour l'inactivation de mycotoxines, en particulier de fumonisines et de dérivés de fumonisine, **caractérisé en ce que** l'additif d'aliment pour animaux contient un microorganisme selon l'une des revendications 1 à 4, en un nombre de germes de 2 x 10⁸/kg d'additif d'aliment pour animaux à 2 x 10¹⁵/kg d'additif d'aliment pour animaux, en particulier de 1 x 10⁹/kg d'additif d'aliment pour animaux à 5 x 10¹²/kg d'additif d'aliment pour animaux.

16. Additif d'aliment pour animaux selon la revendication 15, **caractérisé en ce qu'**il contient en outre au moins une autre bactérie ou une levure choisie parmi Sphingomonas sp. DSM 14170 et DSM 14167, Stenotrophomonas nitritreducens DSM 14168, Stenotrophomonas sp. DSM 14169, Ralstonia eutropha DSM 14171, Eubacterium sp. DSM 14197, Trichosporon mycotoxinivorans DSM 14153, Cryptococcus sp. DSM 14154, Rhodctorula yarrowii DSM 14155, Trichosporon mucoides DSM 14156, Trichosporon dulcitum DSM 14162 ou Eubacterium DSM 11798 pour la détoxification de mycotoxines, en particulier de fumonisines et de dérivés de fumonisine, de zéaralénone ou de dérivés de zéaralénone, d'ochratoxines, de trichothécènes et/ou d'aflatoxines.

17. Utilisation d'un additif d'aliment pour animaux selon la revendication 15 ou 16, pour l'inactivation de la fumonisine B1, B2, B3 et de dérivés de fumonisine, de zéaralénone ou de dérivés de zéaralénone, de zéaralénol, de glycosides de zéaralénone, d'aflatoxines B1, B2, G1, G2, M1, M1, de désoxynivalénol (DON), de toxine T-2, de toxine HT-2, de nivalénol, de monoacétoxyscirpénol, de diacétoxycirpénol, de trichodermol, de verrucarine, de rorodine, d'acétyl-désoxynivalénol, d'isotrichlodermine, d'hydroxy-isotrichodermine, de calonectrine, de tétraol T-2, de triol T-2, de désacétylnéosolaniol, de néosolaniol, d'acétylnéosolaniol, de sporotrichiol, de trichotriol, de sambucinol et de culmorine et/ou d'ochratoxine A, B, C, D.
